Europäisches Patentamt

European Patent Office

Office européen des brevets

(19)

(11) Veröffentlichungsnummer: **0 423 471 A2**

# (12) EUROPÄISCHE PATENTANMELDUNG

(21) Anmeldenummer: 90116532.4

(22) Anmeldetag: 29.08.90

(51) Int. Cl.5: **A61K 7/043**

(30) Priorität: 19.09.89 DE 3931237

(43) Veröffentlichungstag der Anmeldung:
24.04.91 Patentblatt 91/17

(84) Benannte Vertragsstaaten:
AT BE CH DE DK ES FR GB GR IT LI LU NL SE

(71) Anmelder: **Janda, Rita**
**Rheingoldstrasse 4a**
**W-8000 München 19(DE)**

(72) Erfinder: **Janda, Rita**
**Rheingoldstrasse 4a**
**W-8000 München 19(DE)**

(74) Vertreter: **Säger, Manfred, Dipl.-Ing.**
**Säger & Partner Postfach 81 08 09**
**W-8000 München 81(DE)**

(54) **Wasserverdünnbarer Nagellack.**

(57) Die Erfindung betrifft einen wasserverdünnbaren Nagellack. Dieser enthält als Lackbasis eine emulgatorfreie Polyurethan-Polyharnstoff-Dispersion, die durch Reaktion von Diisocyanaten mit Polyestern und anschließende Modifizierung mit Aminen hergestellt wurde. Dabei sind beispielsweise Diisocyanate mit einem Polyester zu Reaktion gebracht worden. Die Diisocyanate können vorher mit einem Stoff mit Emulgatorwirkung umgesetzt worden sein. Das so erhaltene Polyurethan ist anschließend in wäßriger Lösung mit einem Amin modifiziert worden, wobei sich die Polyurethan-Polyharnstoff-Dispersion gebildet hat.

EP 0 423 471 A2

# WASSERVERDÜNNBARER NAGELLACK

Die vorliegende Erfindung betrifft einen wasserverdünnbaren Nagellack, bestehend aus einer emulgatorfreien Polyurethan-Polyharnstoff-Dispersion, die durch Reaktion von Diisocyanaten mit Polyestern und anschließende Modifizierung mit Aminen hergestellt wurde.

Bisher wurde bei den handelsüblichen Dispersionen von Polyurethan-Polyharnstoffen Emulgatoren zur Dispergierung und Stabilisierung der Polymeren in Wasser eingesetzt. Dabei kennen die Emulgatoren auch chemisch in die Polyurethan-Polyharnstoffkette eingebaut werden. Solche emulgator-haltigen Polyurethan-Polyharnstoff-Dispersionen weisen Nachteile auf, wie beispielsweise Wasserempfindlichkeit bzw. Wasserquellbarkeit. Die Wasseraufnahme kann dabei bis zu mehreren Prozenten betragen. Bei der Herstellung von wasserverdünnbaren Nagellacken ist die Wasserempfindlichkeit so hergestellter Polyurethan-Polyharnstoff-Dispersionen ein beträchtlicher Nachteil. Selbst in Kombination mit anderen wäßrigen Dispersionen, wie Polyacrylaten, kann dieser Nachteil nicht vermieden werden.

Es wurde gefunden, daß wäßrige Nagellacke auf Basis emulgatorfreier Polyurethan-Polyharnstoff-Dispersionen eine wesentlich geringere Wasserquellbarkeit und damit eine deutlicher verbesserte Haltbarkeit aufweisen. Demgemäß ist es Aufgabe der vorliegenden Erfindung, Nagellacke mit geringerer Wasserquellbarkeit und verbesserter Haltbarkeit zu schaffen.

Gelöst wird diese Aufgabe durch einen Nagellack gemäß dem Patentanspruch 1. Die Unteransprüche geben bevorzugte Ausgestaltungen der Erfindung wieder.

Die erfindungsgemäßen Nagellacke enthalten als Lackbasis eine emulgator-freie Polyurethan-Polyharnstoff-Dispersion, die durch Reaktion von Diisocyanaten mit Polyestern und anschließende Modifizierung mit Aminen erhalten wurden. Dabei werden beispielsweise Diisocyanate, wie Isophorondiisocyanat oder Hexamethylendiisocyanat mit einem Polyester, beispielsweise Adipinsäurebutandiol-1,4-Polyester zur Reaktion gebracht. Dabei können die Diisocyanate vorher mit einem Stoff mit Emulgatorwirkung, wie beispielsweise Diolsulfonat, umgesetzt werden.

Das so erhaltene Polyurethan wird anschließend in wäßrigen Lösungen mit einem Amin modifiziert, beispielsweise mit Ethylendiamin oder Diethylmethylamin, wobei sich die Polyurethan-Polyharnstoff-Dispersion bildet. Die bei der Herstellung der Polyurethane eingesetzten Polyester können zusätzlich Polyether enthalten, beispielsweise Polyethylenoxid-, Polypropylenoxid-, Polyether- oder Propylenoxidether. Die erfindungsgemäßen Nagellacke können weiterhin folgende Stoffe enthalten :

Verdickungsmittel in einer Menge von etwa 0,1 bis 2,0 Gew.-Teilen. Als Verdickungsmittel kommen insbesondere Polyurethan-Dispersionen in Betracht, die zum Unterschied von den Polyurethan-Dispersionen der Basislackkomponenten keine Filmbildungseigenschafte aufweisen, sondern lediglich verdickende Eigenschaften haben.

Als pH-Regulatoren können beispielsweise Ammoniak, Amine, wie Triethanolamin, Natronlauge und dergleichen, verwendet werden. Sie werden im allgemeinen in einer Menge von 0,1 bis 10 Gew.-Teilen eingesetzt und so dosiert, daß der pH-Wert der entstehenden Mischung bei etwa 7 bis 14, vorzugsweise 7 bis 7,5 liegt.

Als Dispergierungshilfsmittel bzw. Antiabsetzmittel können Stoffe, wie Bentonid, Smektit, Montmorrilonit, pyrogene Kieselsäure und dergleichen verwendet werden. Sie werden in Mengen von 0,1 bis 10 Gew.-Teilen eingesetzt.

Als Oberflächenadditive werden Mittel hinzugefügt, die die Oberflächeneigenschaften verbessern, beispielsweise die Kratzfestigkeit. Solche Mittel sind beispielsweise wäßrige Polyethylendispersionen, Polypropylen- oder Wachsdispersion, die im allgemeinen einen Feststoffgehalt von etwa 30 bis 60 Gew.-% aufweisen. Sie werden in Mengen von 0,1 bis 10 Gew.-Teilen eingesetzt, vorzugsweise in Mengen von 1 bis 6 Gew.-Teilen.

Als Farbstoffe bzw. Pigmente oder Pigmentpräparationen werden die üblicherweise für Nagellacke verwendeten Stoffe eingesetzt, wie organische Farbstoffe, Pigmentpasten, metalloxidbeschichtete Glimmerpigmente und dergleichen. Sie werden in den üblicherweise verwendeten Mengen eingesetzt, im allgemeinen von etwa 0 bis 10 Gew.-Teilen.

Die erfindungsgemäßen Nagellacke können ferner die folgenden Stoffe enthalten:

Wäßrige Emulsionen von Naturharzen oder Pfropfpolymeren von Naturharzen mit Acrylaten mit einem Feststoffgehalt von etwa 30 bis 60 Gew.-%. Als Naturharz ist beispielsweise Schellack geeignet. Die Dispersionen werden in Mengen von 0 bis 20 Gew.-Teilen eingesetzt, vorzugsweise in Mengen von etwa 0 bis 10 Gew.-%.

Lösungsmittelfreie wäßrige Emulsionen von Epoxidharzen mit einem Feststoffgehalt von etwa 30 bis 60 Gew.-%. Sie werden in Mengen von etwa 0 bis 10 Gew.-Teilen, vorzugsweise in Mengen von etwa 0 bis 8 Gew.-Teilen eingesetzt.

Lösungsmittelfreie wäßrige Emulsionen von Polyestern und/oder Alkydharzen mit einem Feststoffgehalt von etwa 30 bis 60 Gew.-% in einer Menge von etwa 0 bis 10 Gew.-Teilen, vorzugsweise von 1 bis 8 Gew.Teilen.

Lösungsmittelfreie wäßrige Melaminharz-Dispersionen mit einem Feststoffgehalt von etw 30 bis 60 Gew.-% in einer. Menge von 0 bis 20 Gew.-Teilen, vorzugsweise von etwa 1 bis 10 Gew.-Teilen, wie beispielsweise Hydroxymethylmethylolmelamin (HMMM).

Lösungsmittelfreie wäßrige Emulsionen von Polyvinylacetat und/oder -propionat mit einem Feststoffgehalt von etwa 30 bis 60 Gew.-% in einer Menge von 0 bis 20 Gew.-Teilen, vorzugsweise von 1 bis 10 Gew.-Teilen.

Für eine wirksame Vernetzung der als Basislacke dienenden Polyurethane, Vinylester, Acrylate, Methacrylate und dergleichen kann ein zusätzliches Vernetzungsmittel hinzugegeben werden, beispielsweise Aciriden. Die Vernetzer werden in Mengen von 0,1 bis 5 Gew.-Teilen vorzugsweise von etwa 0,2 bis 2 Gew.-Teilen bverwendet.

Weiterhin können die üblichen Zusatzstoffe in den üblicherweise verwendeten Mengen eingesetzt werden, wie beispielsweise Wachse, Filmbildungshilfsmittel, Netz- und Dispergiermittel, Füllstoffe und dergleichen.

Als Füllstoffe eignen sich insbesondere Hohlkügelchen, beispielsweise aus Glas, blättchen- oder schuppenförmige Stoffe und dergleichen.

Die erfindungsgemäßen Nagellacke zeichnen sich durch eine gute Haftung, geringe Wasserquellbarkeit und eine gute Abziehbarkeit vom Fingernagel aus.

Die Erfindung wird nachstehend anhand der Beispiele näher erläutert.

Beispiel 1

80 Teile Diolsulfonat (70%-ig) in einem organischen Lösungsmittel werden in 88,6 Teilen Isophorondiisocyanat und 33,6 Teilen Hexamethylendiisocyanat innerhalb von 2 h zur Reaktion gebracht. 116,7 Gew.-Teile dieses Reaktionsproduktes werden mit 400 Gew.-Teilen eines wasserfreien Adipinsäure-butandiol-1,4-Polyesters bei 100°C 2 h umgesetzt. Das so erhaltene Polymer wird auf 70°C abgekühlt.

453 Teile des so erhaltenen Polymers werden in 600 Teilen warmen Wassers einer Temperatur von 50°C unter starkem Rühren dispergiert. Dabei werden 94 Teile warmes Wasser (Temperatur 50°C) und 6 Teile Ethylendiamin langsam zugegeben und 2 h lang gerührt.

Es entsteht eine feinteilige Dispersion. Die Teilchengröße liegt unter 200 nm.

Beispiel 2

50 Teile Diolsulfonat nach Beispiel 1 werden mit 145 Teilen Dicylcohexylpyrrolidon 1 h bei 90° zur Reaktion gebracht.

194 Teile des so erhaltenen Reaktionsprodukts werden mit 400 Teilen eines wasserfreien Adipinsäure-butandiol-1,4-Polyesters, 28 Teilen eines Polyethylenoxid-polypropylenoxid-polyethers und 16 Teilen Propylenoxidethers einer OH-Zahl von 197 bei 120°C 4 h lang umgesetzt. Das so erhaltene Polymer wird auf 70°C abgekühlt.

545 Teile dieses Polymers werden in 600 Teilen warmen Wasser (Temperatur 50°C) unter starkem Rühren dispergiert. Gleichzeitig werden 154 Teile warmes Wasser (50°C) und 6 Teile Ethylendiamin langsam zugegeben.

Beispiel 3

43 Teile Diolsulfonat aus Beispiel 1 werden mit 74,5 Teilen Isophorondiisocyanat und 28 Teilen Hexamethylendiisocyanat bei 90°C 2 h lang umgesetzt.

119 Teile dieses Reaktionsproduktes werden mit 400 Teilen eines wasserfreien Adipinsäurebutandiol-1,4-Polyesters, 28 Teilen Polyethylenoxidpolypropylenoxid-polyether und 16 Teilen eines Propylenoxidethers mit einer OH-Zahl von 197 bei 100°C 2 h lang umgesetzt. Das erhaltene Polymer wird auf 70°C abgekühlt.

500 Teile dieses Polymers werden in 600 Teilen warmen Wasser (50°C) unter starkem Rühren dispergiert. Gleichzeitig werden 146 Teile warmes Wasser (50°C) und 4 Teile Ethylendiamin langsam zugegeben.

Beispiel 4

70 Teile Diolsulfonat aus Beispiel 1 werden mit 180 Teilen Isophorondiisocyanat bei 120°C während 2 h zur Reaktion gebracht.

209 Teile dieses Polymers werden mit 400 Teilen eines wasserfreien Adipinsäure-butandiol-1,4-Polyesters und 12 Teilen n-Butandiol-1,4 bei 100°C über 3 h zur Reaktion gebracht. Das Polymer wird auf 70°C abgekühlt.

540 Teile dieses Polymers werden in 600 Teilen 50°C warmen Wasser unter starkem Rühren dispergiert. Gleichzeitig werden 140 Teile warmes Wasser (50°C) und 10 Teile Diethylmethylamin langsam zugegeben.

Bei allen Beispielen entstehen feinteilige Dispersionen.

## Ansprüche

1. Wasserverdünnbarer Nagellack, bestehend aus einer emulgatorfreien Polyurethan-Polyharnstoff-Dispersion, hergestellt durch Reaktion von Diisocyanaten mit Polyestern und Modfizierung mit Aminen, und gegebenenfalls:
0,1 - 2,0 Gew.-Teilen eines Verdickungsmittels,
0,1 - 10 Gew.-Teilen eines pH-Regulators,
0,1 - 10 Gew.-Telien eines Dispergierungsmittels
0,1 - 10 Gew.-Teilen eines Oberflächenadditivs und
0 - 18 Gew.-Teilen eines Farbstoffs, Pigments, oder einer Pigmentpräparation.

2. Nagellack nach Anspruch 1, gekennzeichnet durch einen Gehalt an 0 bis 20 Gew.-Teilen einer Methacrylat-Dispersion.

3. Nagellack nach Anspruch 1, gekennzeichnet durch einen Gehalt an 0 bis 20 Gew.-Teilen einer wäßrigen Naturharz-Emulsion oder einer wäßrigen Emulsion eines Pfropfenpolymeren aus einem Naturharz mit Acrylsäure oder Methacrylsäure.

4. Nagellack nach Anspruch 1, gekennzeichnet durch einen Gehalt an 0 bis 10 Gew.-Teilen einer lösungsmittelfreien wäßrigen Epoxidharz-Emulsion.

5. Nagellack nach Anspruch 1, gekennzeichnet durch einen Gehalt an 0 bis 10 Gew.-Teilen einer lösungsmittelfreien wäßrigen Emulsion eines Polyesters oder Alkydharzes.

6. Nagellack nach Anspruch 1, gekennzeichnet durch einen Gehalt an 0 bis 20 Gew.-Teilen einer lösungsmittelfreien wäßrigen Melaminharz-Dispersion.

7. Nagellack nach Anspruch 1, gekennzeichnet durch einen Gehalt an 0 bis 20 Gew.-Teilen einer lösungsmittelfreien wäßrigen Emulsion von Polyvinylacetat und/oder -propionat.

8. Nagellack nach Anspruch 1, gekennzeichnet durch einen Gehalt an 0,1 bis 5 Gew.-Teilen eines Vernetzungsmittels.

9. Nagellack nach Anspruch 7, dadurch gekennzeichnet, daß das Vernetzungsmittel aus Aciriden besteht.

10. Nagellack nach Anspruch 1, dadurch gekennzeichnet, daß er von 5 bis 15%, vorzugsweise etwa 10 % $H_2O$ enthält.

11. Nagellack nach Anspruch 1, gekennzeichnet durch einen Gehalt an 0 bis 10 Gew.-Teilen Talkum.

12. Nagellack nach Anspruch 1, gekennzeichnet durch einen Gehalt an 0 bis 100 Gew.-Teilen Polyether.

13. Verfahren zur Herstellung eines Nagellacks nach den Ansprüchen 1 bis 10, dadurch gekennzeichnet, daß eine emulgatorfreie Polyurethan-Polyharnstoff-Dispersion durch Reaktion von Diisocyanaten mit Polyestern und Modifizierung mit Aminen hergestellt wird.